# EUROPÄISCHE PATENTANMELDUNG

(11) **EP 2 636 424 A1**
(43) Veröffentlichungstag der Anmeldung: **11.09.2013**
(21) Anmeldenummer: 12158740.6
(22) Anmeldetag: 09.03.2012
(51) Int. Cl.: A61M 37/00

(54) **Therapeutisches System mit hohlen Mikronadeln**

(71) Anmelder: LTS LOHMANN Therapie-Systeme AG, 56626 Andernach (DE)
(72) Erfinder: Hoffmann, Dr. Hans-Rainer, 56566 Neuwied (DE); Sameti, Dr. Mohammad, 53177 Bonn (DE); Schmitz, Christoph, 56598 Rheinbrohl (DE); Böhm, Rolf, 56271 Kleinmaischeid (DE)

(57) **Zusammenfassung**

Die vorliegende Erfindung betrifft therapeutische Systeme (1) zur Verabreichung mindestens eines Arzneistoffs in oder durch die Haut, umfassend mindestens ein, den mindestens einen Arzneistoff enthaltendes Reservoir (2), eine Vielzahl von hohlen Mikronadeln (3) mit einem sich von der Basis bis zur Spitze oder annähernd bis zur Spitze erstreckenden Durchgang, einen flächigen Träger (11), an dem die Mikronadeln mit ihrer Basis befestigt oder in den die Mikronadeln mit ihrer Basis eingebettet sind, wobei das mindestens eine Reservoir mit zumindest einem Teil der hohlen Mikronadeln derart in Verbindung steht, dass ein Flüssigkeitsanschluss zwischen dem Reservoir und den Durchgängen der mit dem Reservoir in Verbindung stehenden Mikronadeln besteht, wobei das System eine nicht wiederzuverwendende oder nicht wiederverwendbare Vorrichtung (5) umfasst, mit der Druck auf das mindestens eine Reservoir ausgeübt werden kann.

## Beschreibung

Die vorliegende Erfindung betrifft Systeme zur Verabreichung von Arzneistoffen, wobei die Systeme hohle Mikronadeln aufweisen, durch die ein Arzneistoff in die Haut oder durch die Haut eines Patienten verabreicht werden kann.

Im Stand der Technik sind flächenförmige Verbände bekannt, die Mikronadeln aufweisen. Grundsätzlich kann bei diesen Verbänden zwischen Systemen mit massiven Mikronadeln und Systemen mit hohlen Mikronadeln unterschieden werden, die einen Durchgang oder Kanal zur Verabreichung eines Arzneistoffs aufweisen.

So wird beispielsweise in der DE 2 305 989 ein Verband zum Aufbringen auf die Haut offenbart, der in Form eines Kissens ausgebildet ist. Das Kissen weist eine Materialschicht auf, die mit winzigen Fasern versehen ist, welche von mindestens einer Oberfläche der Schicht vorspringen. Beim Aufbringen dieser Oberfläche der Schicht auf die Haut eines Patienten dringen die Fasern in die Haut ein, so dass ein flüssiges Präparat eines pharmakologisch aktiven Materials, sofern ein solches anwesend ist, in den Patienten eindringen kann und im Körper des Patienten verteilt wird.

Das grundlegende Prinzip von therapeutischen Systemen mit hohlen Mikronadeln ist beispielsweise aus der US 6,611,707 B1 bekannt. Das US-Patent US 6,611,707 B1 offenbart Vorrichtungen mit Mikronadeln zur Verabreichung von Wirkstoffen über oder in biologische Gewebe. Die Vorrichtung umfasst ein Substrat ,an dem eine Mehrzahl von hohlen Mikronadeln befestigt oder in das die Mikronadeln integriert sind, sowie zumindest ein Reservoir, welches den Wirkstoff enthält und das mit den Mikronadeln in Verbindung steht. Der Wirkstoff kann entweder durch Drücken mit einem Finger auf das Reservoir oder mit Hilfe einer Spritze aus dem Reservoir gepresst werden.

Therapeutische Systeme mit hohlen Mikronadeln funktionieren im Prinzip wie eine kontinuierliche Injektion. Zur Verabreichung einer Wirkstoff enthaltenden Lösung oder Suspension ist es erforderlich, diese unter kontrollierten Bedingungen mittels Druck durch die hohlen Mikronadeln in oder unter die Haut zu injizieren. Die Verwendung von externen, wiederverwendbaren Systemkomponenten für eine gut kontrollierbare Injektion, beispielsweise Pumpen, erfordert jedoch mindestens eine Zuleitung in Form eines Schlauches. Das beeinträchtigt die Compliance beim Patienten in erheblichem Maß, verglichen mit Systemen, die zur Erzeugung des nötigen Druckes ohne externe Systemkomponenten auskommen (Single-Use-Systeme).

Der vorliegenden Erfindung lag daher die Aufgabe zugrunde, therapeutische Systeme zur Verabreichung mindestens eines Wirkstoffs in oder durch die Haut über hohle Mikronadeln bereitzustellen, bei denen kein manueller Druck auf das System ausgeübt werden muss, die aber auch ohne wiederverwendbare, mindestens eine Zuleitung erfordernde, externe Systemkomponenten zur Erzeugung des für die Verabreichung des Wirkstoffs erforderlichen Druckes auskommen, um den Wirkstoff aus dem System freisetzen zu können.

Diese Aufgabe wird durch die Bereitstellung von therapeutischen Systemen zur Verabreichung mindestens eines Arzneistoffs gelöst, die zur Erzeugung des für die Freisetzung und Verabreichung des mindestens einen Arzneistoffs erforderlichen Druckes aus einem, den mindestens einen Arzneistoff enthaltenden Reservoir ohne wiederzuverwendende, mindestens eine Zuleitung erfordernde, externe Systemkomponenten auskommen und bei denen die Erzeugung des zur Freisetzung des Arzneistoffs benötigten Druckes erst nach dem Anbringen des therapeutischen Systems auf der Haut des Patienten startet.

Gegenstand der vorliegenden Erfindung ist ein therapeutisches System zur Verabreichung mindestens eines Arzneistoffs in oder durch die Haut, umfassend mindestens ein, den mindestens einen Arzneistoff enthaltendes Reservoir, eine Vielzahl von hohlen Mikronadeln, von denen jede Mikronadel einen Schaft, eine Basis an einem Ende des Schafts und eine Spitze am gegenüberliegenden Ende des Schafts aufweist, sowie mindestens einen sich von der Basis bis zur Spitze oder annähernd bis zur Spitze erstreckenden Durchgang, einen flächigen Träger, an dem die Mikronadeln mit ihrer Basis befestigt oder in den die Mikronadeln mit ihrer Basis eingebettet sind, wobei das mindestens eine Reservoir mit zumindest einem Teil der hohlen Mikronadeln derart in Verbindung steht, dass ein Flüssigkeitsanschluss zwischen dem Reservoir und den Durchgängen der mit dem Reservoir in Verbindung stehenden Mikronadeln besteht.

Das System weist zudem mindestens eine nicht wiederzuverwendende beziehungsweise nicht wiederverwendbare Vorrichtung auf, mit der Druck auf das mindestens eine Reservoir ausgeübt werden kann. Die Vorrichtung, mit der Druck auf das mindestens eine Reservoir ausgeübt werden kann, weist keine Zuleitung auf, die die Compliance beim Patienten erheblich beeinträchtigen würde.
Fig. 1A zeigt eine Ausführungsform mit einer osmotischen Kammer als Vorrichtung zur Ausübung von Druck auf ein Reservoir.
Fig. 1B zeigt die in Fig. 1A dargestellte Ausführungsform während oder nach Applikation auf der Haut eines Patienten.
Fig. 2A zeigt eine Ausführungsform mit einem Magneten und einer 'magnetisierbaren Schicht als Vorrichtung zur Ausübung von Druck auf ein Reservoir vor Applikation.
Fig. 2B zeigt die in Fig. 2A dargestellte Ausführungsform während oder nach Applikation auf der Haut eines Patienten.
Fig. 3A zeigt eine Ausführungsform vor Applikation, bei der die Vorrichtung zur Ausübung von Druck auf ein Reservoir ein Bimetall aufweist.
Fig. 3B zeigt die in Fig. 3A dargestellte Ausführungsform während oder nach Applikation auf der Haut eines Patienten.
Fig. 4A zeigt eine Ausführungsform mit einer expandierbaren Schicht vor Applikation, welche mindestens eine Gas erzeugbaren Substanz aufweist, als Vorrichtung zur Ausübung von Druck auf ein Reservoir.
Fig. 4B zeigt die in Fig. 4A dargestellte Ausführungsform während oder nach Applikation auf der Haut eines Patienten.

Das therapeutische System weist eine Vielzahl von hohlen Mikronadeln auf, um den in dem System enthaltenen mindestens einen Arzneistoff über die Haut oder in die Haut eines Patienten abgeben zu können, nachdem das therapeutische System auf die Haut des Patienten aufgebracht wurde. Jede der hohlen Mikronadeln des Systems weist einen langgestreckten Schaft mit zwei Enden auf, das eine Ende des Schafts ist die Basis der Mikronadel, mit der die Mikronadel an dem flächigen Träger befestigt oder mit der die Mikronadel in den flächigen Träger integriert ist. Das der Basis gegenüber liegende Ende des Schafts ist spitz zulaufend ausgestaltet, um ein möglichst leichtes Eindringen der Mikronadel in die Haut zu ermöglichen. Jede hohle Mikronadel weist mindestens einen Durchgang beziehungsweise Kanal oder mindestens eine Bohrung auf, der/die sich von der Basis der Mikronadel bis zur Spitze der Mikronadel oder bis annähernd zur Spitze der Mikronadel erstreckt. Die Durchgänge weisen vorzugsweise einen runden Durchmesser auf. Der Durchmesser jeden Durchgangs beziehungsweise jeder Bohrung ist so groß, dass eine den mindestens einen Arzneistoff enthaltende Zubereitung, vorzugsweise eine den mindestens einen Arzneistoff enthaltende Lösung oder Suspension, durch den mindestens einen Durchgang einer Mikronadel gelangen kann.

Die Mikronadeln können aus unterschiedlichen Materialien hergestellt sein und/oder bestehen, beispielsweise aus einem Metall, einem keramischen Werkstoff, einem Halbleiter, einem organischen Material, einem Polymer oder einem Komposit. Bevorzugte Materialien zur Herstellung der hohlen Mikronadeln sind beispielsweise pharmazeutisch annehmbarer Edelstahl, Gold, Titan, Nickel, Eisen, Zinn, Chrom, Kupfer, Palladium, Platin, Legierungen der genannten Metalle, Silizium, Siliziumdioxid und Polymere. Bei den Polymeren kann es sich um biologisch abbaubare Polymere handeln, vorzugsweise um Polymere von Hydroxysäuren wie Milchsäure und/oder Glykolsäure, Polylactide, Polyglykolide, Polylactide-co-glykolide, und Copolymere mit Polyethylenglykol, Polyanhydride, Poly(ortho)ester, Polyurethane, Polybuttersäure/n, Polyvaleriansäure/n, und Polylactid-co-caprolacton/e. Bei den Polymeren kann es sich auch um nicht biologisch abbaubare Polymere handeln, beispielsweise aus der Gruppe der Polycarbonate, Polyester oder Polyacrylamide. In einer besonderen Ausführungsform bestehen die hohlen Mikronadeln aus einem monokristallinen Material, vorzugsweise aus monokristallinem Silizium.

Die Mikronadeln können einen Schaft mit einem runden Querschnitt oder einen nicht runden Querschnitt aufweisen, beispielsweise mit einen dreieckigen, viereckigen oder einen polygonalen Querschnitt. Der Schaft kann einen Durchgang oder mehrere Durchgänge aufweisen, die von der Nadelbasis bis zur Nadelspitze oder annähernd zur Nadelspitze verläuft/verlaufen. Der Durchmesser einer Mikronadel beträgt üblicherweise zwischen 1 µm und 500 µm, vorzugsweise zwischen 10 µm und 100 µm. Der Durchmesser eines Durchgangs beträgt üblicherweise zwischen 3 µm und 80 µm. Die Länge einer Mikronadel beträgt üblicherweise zwischen 10 µm und 1 mm, vorzugsweise zwischen 100 µm und 500 µm, und besonders bevorzugt zwischen 150 µm und 350 µm. Bei einer besonderen Ausführungsform beträgt die Insertionstiefe der Mikronadeln in die Haut weniger als 150 µm, vorzugsweise weniger als 100 µm, so dass die Mikronadeln nicht in die Dermis eindringen. Durch die Größe der Mikronadeln kann vermieden werden, dass sie Nerven berühren und Schmerz verursachen.

Die hohlen Mikronadeln sind mit ihrer Basis an einem flächigen Träger befestigt oder in einen flächigen Träger integriert. Die hohlen Mikronadeln sind vorzugsweise im Wesentlichen senkrecht zur Fläche des Trägers stehend angeordnet. Die Mikronadeln können regelmäßig oder unregelmäßig angeordnet sein. Eine Anordnung von mehreren Mikronadeln kann Mikronadeln mit unterschiedlichen Querschnittformen, unterschiedlich dimensionierten Durchmessern und/oder unterschiedlichen Längen aufweisen. Die Anordnung aus mehreren Mikronadeln kann ausschließlich hohle Mikronadeln aufweisen. Die Anordnung kann zusätzlich zu den hohlen Mikronadeln auch massive Mikronadeln umfassen.

Das System weist einen flächigen Träger auf. Das bedeutet, dass der Träger im Wesentlichen eine scheibenförmige, plattenförmige oder folienförmige Grundform hat. Der Träger kann eine runde, ovale, dreieckige, viereckige oder polygonale Grundfläche haben. Der Träger kann aus unterschiedlichen Materialien hergestellt sein, beispielsweise aus einem Metall, einem keramischen Werkstoff, einem Halbleiter, einem organischen Material, einem Polymer oder einem Komposit. Als Materialien, die zur Herstellung des Trägers geeignet sind, können Folien oder bahnförmige Materialien genannt werden, beispielsweise mikroporöse Membranen, vorzugsweise aus Polyethylen (PE) oder Polypropylen (PP), oder Diffusions-Membranen, vorzugsweise aus Ethylen-Vinylacetat-Copolymer (EVA) oder Polyurethan (PUR). Geeignete Materialien zur Herstellung des Trägers können aus der Gruppe ausgewählt sein, die Polyester wie Polyethylenterephthalate (PET), Polycarbonate (PC), Polyetherketone (PAEK),̅, Polyethylennaphthalat (PEN), -Polybutylenterephthalate (PBT), die Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC), Polylactat (PLA), und Kunststoffe auf Cellulose-Basis wie Cellulosehydrat oder Celluloseacetat umfasst. Geeignete Materialien zur Herstellung des Trägers können auch aus der Gruppe ausgewählt sein, die Aluminium, Eisen, Kupfer, Gold, Silber, Platin, Legierungen der vorgenannten Metalle und andere pharmazeutisch annehmbare Metallfolien oder mit Metall bedampfte Folien umfasst.

Vorzugsweise ist der Träger aus einem flexiblen Material hergestellt, beispielsweise einem Kunststoff. Ein Träger aus einem flexiblen Material, der flexibel ist, kann sich besser der Hautoberfläche und ihrer Krümmung anpassen als ein Träger aus einem nicht flexiblen Material. Dadurch wird ein besserer Kontakt des Systems mit der Haut erreicht, wodurch die Verlässlichkeit des Systems verbessert wird.

Das System weist mindestens ein Reservoir auf, welches den mindestens einen Arzneistoff in Form einer Arzneistoffzubereitung enthält, vorzugsweise in einer flüssigen Arzneistoffzubereitung. Das System kann somit ein einziges Reservoir aufweisen, das einen Arzneistoff oder mehrere Arzneistoffe in einer Arzneistoffzubereitung enthält. Das System kann aber auch mehrere Reservoire aufweisen, die unterschiedliche Arzneistoffe oder Arzneistoffkombinationen in den Arzneistoffzubereitungen enthalten können. Das mindestens eine Reservoir dient der Aufbewahrung des im System enthaltenen mindestens einen Arzneistoffs vor der Anwendung des Systems. Das mindestens eine Reservoir soll die mindestens eine Arzneistoff-Zubereitung auch vor Verunreinigungen und vor Arzneistoff abbauenden oder seine/ihre Wirksamkeit beeinträchtigenden Einwirkungen schützen.

Das mindestens eine Reservoir steht mit den Durchgängen der hohlen Mikronadeln derart in Verbindung, dass ein Flüssigkeitsanschluss zwischen dem Reservoir und den Durchgängen der mit dem Reservoir in Verbindung stehenden Mikronadeln besteht. Dadurch kann der Inhalt des mindestens einen Reservoirs über die Durchgänge der Mikronadeln aus dem Reservoir heraus aus dem System freigesetzt werden, wenn nach Applikation des Systems auf die Haut eines Patienten Druck auf das mindestens eine Reservoir ausgeübt wird. Die mindestens eine Arzneistoffzubereitung tritt dann an oder in der Nähe der Spitzen der Mikronadeln aus dem System aus und kann in das Zielgewebe eindringen. Üblicherweise ist das mindestens eine Reservoir an einer Oberfläche des flächigen Trägers befestigt, nämlich an der Oberfläche des Trägers, die der Oberfläche des Trägers gegenüber liegt, aus der die Mikronadeln herausragen.

Das mindestens eine Reservoir ist leicht komprimierbar, um dem auf das mindestens eine Reservoir ausgeübten Druck wenig Widerstand entgegenzubringen und somit den Druck auf die in dem mindestens einen Reservoir enthaltene mindestens eine Arzneistoffzubereitung weiterleiten zu können. Gemäß einer Ausführungsform kann das mindestens eine Reservoir in Form eines flexiblen Beutels vorliegen. Derartige Beutel sind einfach herzustellen und können die in dem mindestens einen Reservoir enthaltene Arzneistoffzubereitung unabhängig von der Richtung, aus der Druck auf den Beutel ausgeübt wird, freisetzen.

Gemäß einer anderen Ausführungsform ist das mindestens eine Reservoir aus einem im Wesentlichen biegesteifen Material gefertigt, das eine oder mehrere Seiten des Reservoirs bildet, wobei mindestens eine Seite des Reservoirs mindestens einen flexiblen Bereich aufweist. Vorzugsweise ist die Seite des Reservoirs flexibel oder weist mindestens einen flexiblen Bereich auf, die dem Träger abgewandt ist. Das ist die Seite des Reservoirs, die der Seite des Reservoirs gegenüber liegt, mit der das Reservoir an dem Träger befestigt ist.

Gemäß einer bevorzugten Ausführungsform ist das Reservoir als Kissen oder Ballon ausgestaltet und aus einem elastischen Material hergestellt, beispielsweise aus einem elastomeren Polymer oder Gummi. Beispiele für Polymere sind Polyester wie Polyethylenterephthalate (PET), Polycarbonate (PC), Polyetherketone (PAEK), Polyethylennaphthalat (PEN), Polybutylenterephthalate (PBT), die Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC). Polylactat (PLA), und Kunststoffe auf Cellulose-Basis wie Cellulosehydrat oder Celluloseacetat.

Das System kann ein einziges Reservoir aufweisen, das mit allen oder nur einem Teil der hohlen Mikronadeln in Verbindung steht. Bei einer anderen Ausführungsform kann das System mehrere Reservoire aufweisen, wobei jedes der Reservoire mit allen hohlen Mikronadeln oder nur mit einem Teil der hohlen Mikronadeln in Verbindung steht.

Das therapeutische System umfasst mindestens einen Arzneistoff, der mit dem System in die Haut oder durch die Haut eines Patienten verabreicht werden kann. Das therapeutische System ist nicht auf die Verabreichung eines bestimmten Arzneistoffs beschränkt. Vielmehr kann nahezu jeder beliebige Arzneistoff allein oder in Kombination mit einem oder mehreren anderen Arzneistoffen, und gegebenenfalls in Kombination mit mindestens einem pharmazeutisch annehmbaren Hilfsstoff mit Hilfe des erfindungsgemäßen therapeutischen Systems verabreicht werden.

Der mindestens eine Arzneistoff liegt in dem mindestens einen Reservoir in Form einer Arzneistoffzubereitung vor. Die Arzneistoffzubereitung ist vorzugsweise eine Flüssigkeit, insbesondere eine Lösung, eine Dispersion oder eine Suspension des mindestens einen Arzneistoffs in einem Lösungsmittel, Dispergier- oder Suspendiermedium. Die Arzneistoffzubereitung kann neben dem mindestens einen Arzneistoff auch weitere pharmakologisch annehmbare Hilfsstoffe enthalten, beispielsweise Substanzen zur Regulierung des pH-Werts, zur Einstellung der Viskosität der Arzneistoffzubereitung, damit die Freisetzungsrate der Arzneimittelzubereitung aus einem gegebenen System eingestellt werden kann, und dergleichen.

Das System kann ein Überpflaster auf der Seite aufweisen, die der Haut nach Applikation des Systems abgewandt ist. Das Überpflaster schützt das System vor nachteiligen Einwirkungen von außen. Es kann der Befestigung des Systems auf der Haut eines Patienten dienen, und das Überpflaster kann als Widerlager für die Vorrichtung zum Ausüben von Druck auf das mindestens eine Reservoir dienen, insbesondere bei Ausführungsformen, bei denen die Vorrichtung mindestens ein expandierbares Element, beispielsweise eine osmotische Kammer oder ein expandierbares Kissen mit einer gaserzeugenden Substanz, umfasst. Das Überpflaster kann das mindestens eine Reservoir, den Träger mit hohlen Mikronadeln und die mindestens eine Vorrichtung zum Ausüben von Druck auf das mindestens eine Reservoir an mindestens einer Seite, vorzugsweise an zwei einander gegenüber liegenden Seiten, und besonders bevorzugt allseitig überragen.

Bei einer Ausführungsform ist das Überpflaster fest mit der Vorrichtung zum Ausüben von Druck auf das Reservoir fest verbunden. Dadurch ist eine besonders wirkungsvolle Ableitung des Druckes auf das mindestens eine Reservoir möglich.

Das Überpflaster ist vorzugsweise ein flächiges, folienförmiges Element. Das Überpflaster kann aus dem gleichen Material wie der Träger gefertigt sein. Vorzugsweise umfasst das Überpflaster einen Abschnitt einer Polymerfolie. Das Material der Polymerfolie kann aus einer Gruppe unterschiedlicher Polymere ausgewählt sein, mit denen sich Folien mit den gewünschten Eigenschaften herstellen lassen. Beispielsweise kann die Polymerfolie des Überpflasters durchlässig für Wasser oder Wasserdampf sein. Dadurch wird der Zutritt von Feuchtigkeit zur Vorrichtung zum Ausüben von Druck auf das Reservoir ermöglicht. Bei anderen Ausführungsformen kann die Polymerfolie des Überpflasters wasserdampfundurchlässig sein. Je nach Dicke und Material der Polymerfolie des Überpflasters lässt sich die Elastizität des Überpflasters und somit des System beeinflussen.

Dem Grunde nach kommt jedes physiologisch annehmbare Polymer als Material für die Polymerfolie des Überpflasters in Betracht. Geeignete Materialien zur Herstellung der Polymerfolie können aus der Gruppe ausgewählt sein, die Polyester wie Polyethylenterephthalate (PET), Polycarbonate (PC), Polyetherketone (PAEK), Polyehtylennaphthalat (PEN), Polybutylenterephthalate (PBT), die Polyurethane (PU), Polystyrole (PS), Polyamide (PA), Polyoxymethylen (POM), Polyolefine wie Polyethylen (PE) und Polypropylen (PP), Polytetrafluorethylen (PTFE), Polyvinylchlorid (PVC), Polyvinylidenchlorid (PVDC), Polylactat (PLA), und Kunststoffe auf Cellulose-Basis wie Cellulosehydrat oder Celluloseacetat umfasst. Es ist auch möglich ein Gewebe anstatt einer Folie für das Überpflaster zu verwenden.

Das Überpflaster kann auf seiner Oberfläche, die bei Applikation des Systems der Haut zugewandt ist, mit einer haftklebenden Schicht versehen sein. Mit dieser haftklebenden Schicht kann das Überpflaster fest mit der Vorrichtung zum Ausüben von Druck auf das mindestens eine Reservoir befestigt sein. Die haftklebende Schicht erleichtert das Applizieren des Systems auf der Haut eines Patienten und stellt zudem den Halt des Systems auf der Haut sicher.

Gemäß einer anderen Ausführungsform kann das System mit Hilfe eines haftklebenden Klebestreifens auf der Haut eines Patienten befestigt werden. Dieser Klebestreifen kann zusätzlich zu oder anstatt des Überpflasters verwendet werden.

Gemäß einer anderen oder zusätzlichen Ausführungsform kann die Oberfläche des Trägers, die bei Applikation des Systems mit der Haut des Patienten in Berührung kommt, eine haftklebende Schicht aufweisen. Diese haftklebende Schicht erleichtert das Befestigen des Systems auf der Haut des Patienten und stellt den Halt des Systems auf der Haut eines Patienten sicher. Die haftklebende Schicht der Trägers umgibt die Schäfte der Mikronadeln, ohne die Öffnungen der Durchgänge an oder bei den Spitzen der Mikronadeln zu verschließen.

In einer anderen oder zusätzlichen Ausführungsform weisen die Mikronadeln oder zumindest ein Teil der Mikronadeln eine Vielzahl von Strukturen auf, die zum Eindringen in die Haut geeignet sind und das Herausziehen aus der Haut erschweren, indem die zum Einstechen der Mikronadeln in die Haut erforderliche Kraft geringer ist als die Kraft zum Herausziehen der Mikronadeln aus der Haut. Vorzugsweise sind die Strukturen als Widerhaken oder als Hinterschneidungen der Schäfte ausgebildet.

Das therapeutische System weist eine nicht wiederzuverwendende oder nicht wiederverwendbare Vorrichtung auf, mit der Druck auf das mindestens eine Reservoir ausgeübt werden kann, nachdem das System appliziert wurde. Der besondere Vorteil dieser Systeme liegt neben anderen Vorteilen auch darin, dass der Druck auf das mindestens eine Reservoir erst nach dem Anbringen des Systems an der Haut eines Patienten ausübbar ist.

Gemäß einer Ausführungsform ist die Vorrichtung zum Ausüben von Druck auf das mindestens eine Reservoir als osmotische Kammer ausgebildet. Die osmotische Kammer umfasst eine semipermeable Wandung. Der Begriff "semipermeabel" bedeutet, dass das Material für Wasser durchlässig, für die in dem Wasser gelösten Substanzen jedoch undurchlässig ist. Bei einer Ausführungsform kann die gesamte Wandung der osmotischen Kammer aus einem semipermeablen Material bestehen. Bei anderen Ausführungsformen kann nur eine Seite oder ein Bereich der Wandung der osmotischen Kammer aus einem semipermeablen Material bestehen. Die Wandung der osmotischen Kammer besteht aus einem flexiblen Material oder weist zumindest einen Bereich aus einem flexiblen Material auf, wobei dieser flexible Bereich mit dem mindestens einen Reservoir bzw. dem mindestens einen flexiblen Bereich des mindestens einen Reservoirs Kontakt in kommt, wenn sich die osmotische Kammer ausdehnt.

Die osmotische Kammer enthält mindestens eine osmotisch wirksame Substanz. Bei der osmotisch wirksamen Substanz kann es sich um ein Salz, ein Kohlenhydrat, vorzugsweise um ein Mono- oder Disaccharid, um Glycerin, einem Superabsorber, oder deren Mischungen handeln.

Unter einem Superabsorber werden Kunststoffe verstanden, die ein Vielfaches ihres Eigengewichts - bis zum 1000-fachen - an Flüssigkeit aufzusaugen, insbesondere Wasser oder eine wässrige Lösung. Bei den bevorzugten Superabsorbern handelt es sich um Copolymere aus Acrylsäure und Natriumacrylat, bei denen die Polymermoleküle miteinander vernetzt sind wobei das Verhältnis der beiden Monomere zueinander variieren kann. Unter dem Begriff "osmotisch wirksame Substanz" werden im Sinne der vorliegenden Erfindung auch Lösungen oder Suspensionen verstanden, die osmotisch wirksam sind und beispielsweise eine oder mehrere der vorgenannten Substanzen enthalten. Vorzugsweise ist die osmotisch wirksame Substanz nicht toxisch und nicht reizend, sondern biokompatibel und pharmakologisch annehmbar. Die osmotisch wirksame Substanz ist in der osmotischen Kammer in einer Menge enthalten, die ausreicht, einen hinreichend hohen Druck auf das mindestens eine Reservoir auszuüben, damit die in dem mindestens einen Reservoir enthaltene Arzneistoffzubereitung in therapeutisch ausreichender Menge aus dem System freigesetzt werden kann.

Die osmotisch wirksame Substanz in der osmotischen Kammer zieht nach Applikation des Systems auf die Haut eines Patienten Wasser in die Kammer. Je nach Ausgestaltung des Systems kann es sich bei dem Wasser um von der Haut abgegebene Feuchtigkeit handeln. Bei dieser Ausgestaltung ist das Überpflaster vorzugsweise wasserdampfundurchlässig. Bei einer anderen oder zusätzlichen Ausgestaltung kann das System unmittelbar vor oder nach seiner Applikation auf der Haut eines Patienten mit Wasser befeuchtet werden. Bei dieser Ausgestaltung ist das Überpflaster für Wasser durchlässig. Somit kann das auf das applizierte System aufgebrachte Wasser durch das Überpflaster und über die semipermeable Wandung in die osmotische Kammer eindringen. Dadurch steigt der Druck in der osmotischen Kammer. Aufgrund der Elastizität der Kammerwandung nimmt die osmotische Kammer an Volumen zu, denn die osmotische Kammer weist keine Öffnung auf, durch die das einströmende Wasser oder die Lösung austreten könnte. Durch die Volumenzunahme der osmotischen Kammer übt diese einen Druck auf das mindestens eine Reservoir aus. Das mindestens eine Reservoir wird dadurch komprimiert und die in dem mindestens einen Reservoir enthaltene Arzneistoffzubereitung wird aus seinem Reservoir in die Durchgänge der hohlen Mikronadeln herausgedrückt. Über die Durchgänge in den hohlen Mikronadeln wird die Arzneistoffzubereitung aus dem System freigesetzt.

Gemäß einer anderen Ausführungsform weist die Vorrichtung zum Ausüben von Druck auf das mindestens eine Reservoir einen Magneten oder Elektromagneten sowie eine magnetische oder magnetisierbare Schicht auf. Bei dieser Ausführungsform ist das mindestens eine Reservoir zwischen dem Magneten oder Elektromagneten und der magnetischen oder magnetisierbaren Schicht angeordnet. Die magnetische oder magnetisierbare Schicht kann auf der Oberfläche des Trägers angeordnet sein, die dem Reservoir zugewandt ist, so dass die magnetische oder magnetisierbare Schicht zwischen dem Träger und dem mindestens einen Reservoir liegt. Bei einer alternativen oder zusätzlichen Ausgestaltung kann die magnetische oder magnetisierbare Schicht auch auf der gegenüberliegenden Oberfläche des Trägers angeordnet sein, d. h. auf der Oberfläche des Trägers, an der die Mikronadeln angeordnet sind bzw. von der die Mikronadeln abstehen. Unter einer "magnetisierbaren Schicht" ist eine Schicht zu verstehen, die aus einem Material besteht oder ein Material enthält, welches bei Anlegen eines magnetischen Feldes magnetisch wird, beispielsweise eine Schicht aus oder mit einem ferromagnetischen oder ferrimagnetischen Material.

Durch die magnetische Anziehungskraft zwischen Magnet oder Elektromagnet - sofern ein elektrischer Strom durch den Elektromagneten fließt - und der magnetischen bzw. magnetisierbaren Schicht wird das dazwischen angeordnete mindestens eine Reservoir zusammengedrückt. Durch den sich dadurch im Inneren des Reservoirs erhöhenden Druck wird die Arzneistoffzubereitung über die Durchgänge in den hohlen Mikronadeln freigesetzt. Durch Wahl der Stromstärke oder den Materialien für den Magneten und/oder die magnetische oder magnetisierbare Schicht kann die magnetische Anziehungskraft zwischen (Elektro-)Magnet und magnetischer/magnetisierbarer Schicht eingestellt werden. Bei einer gegebenen Kombination aus Reservoir, Träger mit hohlen Mikronadeln und Arzneizubereitung kann durch Einstellung der magnetischen Anziehungskraft die Freisetzungsgeschwindigkeit der Arzneizubereitung aus dem System eingestellt werden.

Mit Hilfe mindestens eines Abstandshalters zwischen dem Magneten und der magnetischen oder magnetisierbaren Schicht kann verhindert werden, dass Druck auf das Reservoir ausgeübt wird, bevor das System zur Anwendung kommt. Nach dem Anbringen des Systems auf der Haut eines Patienten kann der Abstandshalter entfernt werden, so dass sich Magnet und magnetische bzw. magnetisierbare Schicht anziehen und der für die Freisetzung des Arzneistoffs vorgesehene Druck auf das Reservoir ausgeübt werden kann.

Bei einer anderen Ausgestaltung der vorgenannten Ausführungsform kann vorgesehen werden, dass der Magnet erst nach Anbringen des die anderen Bestandteile des Systems, d.h. den Träger mit den Mikronadeln, das Reservoir, die magnetische oder magnetisierbare Schicht und gegebenenfalls das Überpflaster, umfassenden Elements aufgebracht wird, so dass dadurch das System vervollständigt und der Druck auf das Reservoir zur Freisetzung des Arzneistoffs ausgeübt wird.

Bei Verwendung eines Elektromagneten kann das System ein galvanisches Element aufweisen, mit dem eine elektrische Spannung an den Elektromagneten angelegt und durch Schließen des elektrischen Stromkreises ein Strom erzeugt werden kann. Das galvanische Element, beispielsweise eine Batterie, vorzugsweise eine so genannte Knopfzelle, kann unmittelbar vor oder nach dem Anbringen des Systems auf der Haut eines Patienten in eine dafür vorgesehene Öffnung eingeführt werden. Der Stromkreis kann bereits durch das Einführen des galvanischen Elements in die Öffnung geschlossen werden, so dass bereits mit dem Einführen des galvanischen Elements in die Öffnung der für die Freisetzung des Arzneistoffs aus dem System erforderliche Druck auf das mindestens eine Reservoir erzeugt wird. In einer anderen Ausgestaltung kann der Stromkreis einen Schalter aufweisen, der unabhängig vom Einsetzen des galvanischen Elements in die Öffnung den Stromkreis schließen kann. Bei dieser Ausgestaltung ist es nicht erforderlich, das galvanische Element unmittelbar vor oder nach dem Befestigen des Systems auf der Haut eines Patienten anzuschließen. Vielmehr kann das galvanische Element bereits integraler Bestandteil des therapeutischen Systems sein, das nach seiner Befestigung auf der Haut des Patienten einfach eingeschaltet wird. Vorteilhafterweise kann ein System dieser Ausführung auch einfach wieder abgeschaltet werden, um die Arzneistofffreisetzung zu unterbrechen oder zu beenden.

Gemäß einer anderen Ausführungsform weist die Vorrichtung zum Ausüben von Druck auf das mindestens eine Reservoir eine Platte oder mindestens einen Streifen aus einem Bimetall auf. Ein Bimetall ist ein Metallstreifen aus zwei Schichten unterschiedlicher Metalle mit unterschiedlichem Längenausdehnungskoeffizienten, die miteinander stoffschlüssig oder formschlüssig verbunden sind. Charakteristisch ist die Verbiegung des Streifens bei Temperaturänderung. Bei einer bevorzugten Ausgestaltung ist das mindestens eine Reservoir in einem Kompartiment angeordnet, das eine Wand aus einem Bimetall oder mehreren, gegebenenfalls miteinander verbundenen Bimetall-Streifen aufweist. Die aus einem Bimetall bestehende Wand oder die den mindestens einen Bimetall-Streifen aufweisende Wand des Kompartiments ist vorzugsweise die Wand des Kompartiments, die dem Träger abgewandt und dem Überpflaster zugewandt ist.

Diese Ausführungsform ermöglicht eine von Wärme abhängige Druckausübung auf das mindestens eine Reservoir. Bei zunehmender Erwärmung krümmt sich das Bimetall stärker und übt somit einen stärkeren Druck auf das mindestens eine Reservoir aus. Bei einer bevorzugten Ausgestaltung ist das Bimetall bei Lagertemperatur im Wesentlichen lang gestreckt und krümmt sich bei steigender Temperatur. Die bevorzugte Lagertemperatur für diese Ausführungsformen des Systems kann beispielsweise etwa 4°C betragen. Bei anderen Systemen dieser Ausführungsform kann die bevorzugte Lagertemperatur Raumtemperatur sein, d. h. etwa zwischen 19°C und 25°C, vorzugsweise zwischen 21 °C und 23°C. Der Vorteil einer niedrigeren Lagertemperatur besteht in der größeren Temperaturdifferenz zur Temperatur der Haut. Bei einer höheren Temperaturdifferenz ist die Biegung des Bimetalls höher, so dass durch eine niedrigere Lagertemperatur ein höherer Druck durch das Bimetall auf das Wirkstoffreservoir ausgeübt werden kann, wenn das System angewendet wird.

Durch das Applizieren des Systems auf der Haut eines Patienten wird das System über die Körpertemperatur des Patienten erwärmt. Durch die Erwärmung des Systems biegt sich das Bimetall und übt einen Druck auf das darunter liegende Reservoir aus. Das mindestens eine Reservoir wird dadurch komprimiert und die in dem mindestens einen Reservoir enthaltene Arzneistoffzubereitung wird aus seinem Reservoir in die Durchgänge der hohlen Mikronadeln herausgedrückt. Über die Durchgänge in den hohlen Mikronadeln wird die Arzneistoffzubereitung aus dem System freigesetzt

Gemäß einer anderen Ausführungsform umfasst die Vorrichtung zum Ausüben von Druck auf das mindestens eine Reservoir ein expandierbares Element mit mindestens einer zur Gaserzeugung befähigten Substanz auf. Das expandierbare Element ist als Schicht zwischen dem Überpflaster des Systems und dem mindestens einen Reservoir angeordnet. Bei dem gaserzeugenden Element handelt es sich vorzugsweise um einen allseitig geschlossenen Beutel, besonders bevorzugt um einen allseitig geschlossenen Beutel, der zwei randständig miteinander verbundenen gasundurchlässigen Schichten aufweist. Das expandierbare Element enthält mindestens eine zur Gaserzeugung befähigte Substanz, die bei Kontakt mit einem Lösungsmittel, vorzugsweise mit Wasser, Gas erzeugen kann.

Bei einer Ausgestaltung kann die Wand oder zumindest ein Teil der Wand des expandierbaren Elements aus einem für das Lösungsmittel, beispielsweise für Wasser oder Wasserdampf, durchlässiges aber für Gas undurchlässiges Material bestehen. Dadurch kann das Lösungsmittel, beispielsweise von der Haut abgegebene Feuchtigkeit oder gesondert zugeführte Feuchtigkeit beziehungsweise gesondert zugeführtes Lösungsmittel in das expandierbare Element eindringen und mit der darin enthaltenen, zur Gaserzeugung befähigten Substanz reagieren und so ein Gas erzeugen. In Folge der Gasentwicklung expandiert das Element und übt Druck auf das mindestens eine Reservoir aus.

Bei einer anderen Ausgestaltung kann das für die Gaserzeugung erforderliche Lösungsmittel, beispielsweise Wasser, in Form eines oder mehrerer das Lösungsmittel umhüllenden Kompartimente in dem Lumen des expandierbaren Elements bereitgestellt werden, so dass das Lösungsmittel nicht mit der zur Gaserzeugung befähigten Substanz in Kontakt kommt. Bei dieser Ausgestaltung ist die Wandung des expandierbaren Elements sowohl für das Lösungsmittel wie auch für Gas undurchlässig. Durch Zerstören der mit Lösungsmittel gefüllten Kompartimente unmittelbar vor oder nach Applikation des Systems auf der Haut eines Patienten, beispielsweise durch manuelles Reiben auf dem System, Drücken oder Brechen des Systems, kann das Lösungsmittel aus den es umhüllenden Kompartimenten freigesetzt werden und mit der zur Gaserzeugung befähigten Substanz reagieren. Durch die Volumenzunahme des expandierbaren Elements übt dieses einen Druck auf das mindestens eine Reservoir aus. Das mindestens eine Reservoir wird dadurch komprimiert und die in dem mindestens einen Reservoir enthaltene Arzneistoffzubereitung wird aus seinem Reservoir in die Durchgänge der hohlen Mikronadeln herausgedrückt. Über die Durchgänge in den hohlen Mikronadeln wird die Arzneistoffzubereitung aus dem System freigesetzt

Bei einer anderen Ausgestaltung der Ausführungsform, bei der der Arzneistoff mit Hilfe der Erzeugung von Gas aus dem Reservoir freigesetzt wird, kann die Gaserzeugung mit Hilfe micropyrotechnischer Elemente erfolgen. Unter Micropyrotechnik ist ein Mikrosystem zu verstehen, das ähnlich einem Mikrochip aus einem Substrat besteht, und der mindestens mit einer Steuereinheit, einer Zündvorrichtung und einer zur Gaserzeugung fähigen Schicht ausgerüstet ist. Dieses Mikrosystem befindet sich in oder an einem expandierbaren Element, in das das nach der Zündung des Mikrosystems erzeugte Gas entweicht und damit das expandierbare Element "aufbläst".

Die Vorteile der Verwendung von Micropyrotechnik zur Erzeugung von Gas in Ausführungsformen des erfindungsgemäßen Systems liegen in der Materialersparnis für das System und der damit verbundenen Kostenersparnis, der Möglichkeit, sehr kleine Bauformen zu realisieren. Zudem sind eine präzise Steuerung und eine definierte Gaserzeugung pro Zeit möglich.

Die erfindungsgemäßen therapeutischen Systeme sind einfach in ihrer Anwendung, sie genießen eine hohe Akzeptanz bei Patienten, weil sie keine mit externen Zuleitungen versehene Pumpen oder dergleichen aufweisen, und der für die Freisetzung des mindestens einen Arzneistoffs aus dem System erforderliche Druck wird erst bei oder unmittelbar nach der Applikation des Systems aufgebaut.

Die Erfindung erstreckt sich auch auf die Verwendung des Systems zur Verabreichung mindestens eines Arzneistoffs, insbesondere zur Verabreichung des mindestens einen Arzneistoffs in therapeutisch ausreichender Menge.

Nachfolgend wird die Erfindung an Hand konkreter Ausführungsbeispiele und mit Bezug auf die Figuren näher erläutert. Dabei dienen die Ausführungsbeispiele und die Figuren lediglich der Veranschaulichung der Erfindung, ohne diese jedoch in irgendeiner Weise einzuschränken.

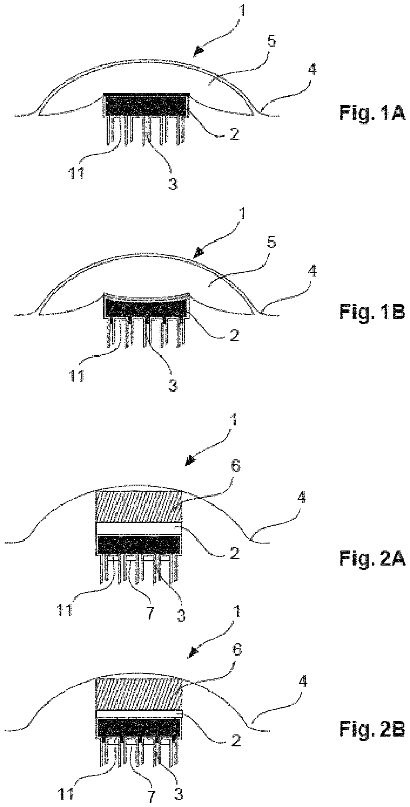

Fig. 1A zeigt eine schematische Darstellung des Systems 1 mit einer osmotischen Kammer 5 als Vorrichtung zum Ausüben von Druck auf das Arzneistoff enthaltende Reservoir 2 im Längsschnitt. Das System 1 weist einen Träger 11 mit hohlen Mikronadeln 3 auf, die sich im Wesentlichen senkrecht zur Fläche des Trägers 11 erstrecken. Der Träger 11 ist mit einem Reservoir 2 verbunden, welches eine Arzneistoffzubereitung enthält. Ferner weist das System 1 eine osmotische Kammer 5 auf, die mindestens eine osmotisch wirksame Substanz enthält. Zudem umfasst das System 1 ein Überpflaster 4. In der Schnittzeichnung überragt das Überpflaster 4 die osmotische Kammer 5 beidseitig, wobei die osmotische Kammer 5 das Reservoir 2 ebenfalls beidseitig überragt. Fig. 1A zeigt das System 1 im Zustand vor seiner Applikation auf die Haut eines Patienten. Das in Fig. 1A dargestellte System 1 wird in Fig. 1B nach oder während der Applikation gezeigt. Hierbei veranschaulicht der Pfeil die Freigaberichtung des Arzneistoffs. Durch die in die osmotische Kammer 5 eindringende Feuchtigkeit dehnt sich die osmotische Kammer 5 aus, drückt auf das

Reservoir 2 und presst somit die Arzneizubereitung über die hohlen Mikronadeln 3 aus dem System 1.

Fig. 2A und 2B zeige eine alternative Ausführungsform des Systems 1, bei der die Vorrichtung zum Ausüben von Druck auf das Reservoir 2 einen Elektromagneten 6, der zwischen Reservoir 2 und Überpflaster 3 angeordnet ist, und eine magnetisierbare Schicht 7 umfasst, welche auf der Oberfläche des Trägers 11 angeordnet ist, die dem Reservoir 2 abgewandt ist. In Fig. 2A ist das System 1 vor seiner Applikation dargestellt. Es fließt kein Strom durch den Elektromagneten 6, so dass es keine magnetische Anziehungskraft zwischen dem Elektromagneten 6 und der magnetisierbaren Schicht 7 gibt. Fig. 2B veranschaulicht das in Fig. 2A dargestellte System 1 während seiner Anwendung am Patienten. Durch eine elektrisch leitende Verbindung des Elektromagneten 6 mit einer Stromquelle über einen geschlossenen Stromkreis fließt elektrischer Strom durch den Elektromagneten 6, wodurch die magnetisierbare Schicht 7 vom Elektromagneten angezogen wird. Das zwischen Elektromagneten 6 und magnetisierbarer Schicht 7 angeordnete Reservoir 2 wird dadurch zusammengepresst. In Folge dessen steigt der Druck im Inneren des Reservoirs 2 und die Arzneistoffzubereitung kann in Richtung des Pfeils aus dem System 1 herausgedrückt werden.

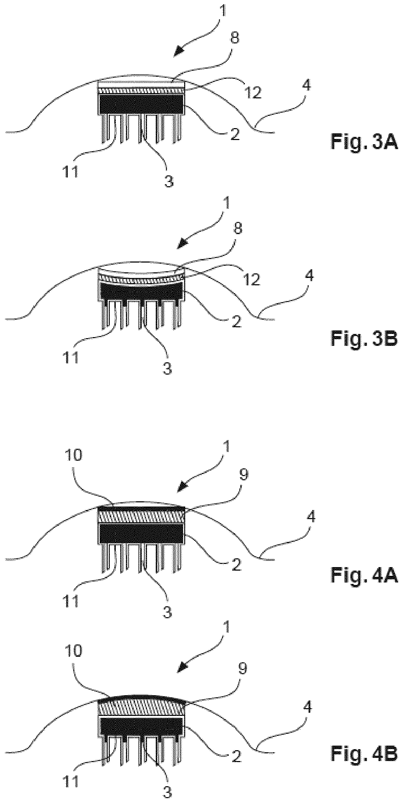

Fig. 3A und Fig. 3B zeigen eine Ausführungsform des Systems 1 im Längsschnitt, bei dem das System 1 einen Träger 11 mit hohlen Mikronadeln 3 und einem Reservoir 2 umfasst. Das Reservoir 2 ist in einem Kompartiment 12 angeordnet, das eine Wandung aus einem Bimetall 8 aufweist, welche vom Überpflaster 4 überdeckt wird. Durch die zunehmende Erwärmung des Systems 1 nach seiner Applikation auf der Haut eines Patienten krümmt sich das Bimetall 8 stärker und drückt auf das Reservoir 2, so dass die Arzneistoffzubereitung aus dem System 1 freigesetzt werden kann.

Fig. 4A zeigt eine Ausführungsform mit einem expandierbaren Element 9, das eine gaserzeugende Komponente 10 enthält. Die gaserzeugende Komponente 10 kann in die Wandung des expandierbaren Elements 9 integriert sein, kann als partikuläre Zubereitung im Inneren des expandierbaren Elements 9 vorliegen, oder als folienförmige Zubereitung. Durch die Erzeugung von Gas expandiert das expandierbare Element 9 und dehnt sich aus. Dadurch wird Druck auf das Reservoir 2 ausgeübt und die im Reservoir 2 enthaltene Arzneistoffzubereitung wird über die hohlen Mikronadeln 3 aus dem System 1 herausgedrückt.

## Patentansprüche

1. Therapeutisches System zur Verabreichung mindestens eines Arzneistoffs in oder durch die Haut, umfassend mindestens ein, den mindestens einen Arzneistoff enthaltendes Reservoir, eine Vielzahl von hohlen Mikronadeln, von denen jede Mikronadel einen Schaft, eine Basis an einem Ende des Schafts und eine Spitze am gegenüberliegenden Ende des Schafts aufweisen, sowie mindestens einen sich von der Basis bis zur Spitze oder annähernd bis zur Spitze erstreckenden Durchgang, einen flächigen Träger, an dem die Mikronadeln mit ihrer Basis befestigt oder in den die Mikronadeln mit ihrer Basis eingebettet sind, wobei das mindestens eine Reservoir mit zumindest einem Teil der hohlen Mikronadeln derart in Verbindung steht, dass ein Flüssigkeitsanschluss zwischen dem Reservoir und den Durchgängen der mit dem Reservoir in Verbindung stehenden Mikronadeln besteht,
**dadurch gekennzeichnet, dass**
das System eine nicht wiederzuverwendende oder nicht wiederverwendbare Vorrichtung umfasst, mit der Druck auf das mindestens eine Reservoir ausgeübt werden kann.

2. Therapeutisches System nach Anspruch 1, **dadurch gekennzeichnet, dass** das Reservoir komprimierbar ist und aus einem flexiblen Material besteht oder mindestens einen flexiblen Bereich auf mindestens einer seiner Seiten aufweist.

3. Therapeutisches System nach Anspruch 1 oder 2, **dadurch gekennzeichnet, dass** es ein Überpflaster aufweist.

4. Therapeutisches System nach einem der vorhergehenden Ansprüche, **dadurch gekennzeichnet, dass** die Seite des Trägers, an der die hohlen Mikronadeln angeordnet sind, eine haftklebende Schicht aufweist.

5. Therapeutisches System nach einem der vorhergehenden Ansprüche, **dadurch gekennzeichnet, dass** die hohlen Mikronadeln mit Widerhaken versehen sind.

6. Therapeutisches System nach einem der vorhergehenden Ansprüche, **dadurch gekennzeichnet, dass** die Vorrichtung zur Ausübung von Druck auf das mindestens eine Reservoir als osmotische Kammer ausgebildet.

7. Therapeutisches System nach einem der Ansprüche 1 bis 5, **dadurch gekennzeichnet, dass** die Vorrichtung zur Ausübung von Druck auf das mindestens eine Reservoir einen Magneten oder Elektromagneten sowie eine magnetische oder magnetisierbare Schicht umfasst.

8. Therapeutisches System nach einem der Ansprüche 1 bis 5, **dadurch gekennzeichnet, dass** die Vorrichtung zur Ausübung von Druck auf das mindestens eine Reservoir eine Platte oder mindestens einen Streifen aus einem Bimetall aufweist.

9. Therapeutisches System nach einem der Ansprüche 1 bis 5, **dadurch gekennzeichnet, dass** die Vorrichtung zur Ausübung von Druck auf das mindestens eine Reservoir ein expandierbares Element aufweist, das mindestens eine zur Erzeugung von Gas befähigte Substanz enthält.

10. Verwendung eines Systems nach einem der vorhergehenden Ansprüche zur Verabreichung mindestens eines Arzneistoffs.
